(19) Europäisches Patentamt
European Patent Office
Office européen des brevets

(11) **EP 4 134 064 A1**

(12) **EUROPEAN PATENT APPLICATION**

(43) Date of publication:
**15.02.2023 Bulletin 2023/07**

(21) Application number: **21193322.1**

(22) Date of filing: **26.08.2021**

(51) International Patent Classification (IPC):
*A61K 8/02* *(2006.01)*          *A61K 8/26* *(2006.01)*
*A61K 8/96* *(2006.01)*          *A61K 8/9789* *(2017.01)*
*A61Q 19/00* *(2006.01)*

(52) Cooperative Patent Classification (CPC):
**A61K 8/0212; A61K 8/0225; A61K 8/26;**
**A61K 8/965; A61K 8/9789; A61Q 19/00**

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(30) Priority: **13.08.2021 KR 20210107351**

(71) Applicant: **Macsumsuk GM Co., Ltd.**
**Gyeongsangbuk-do 38911 (KR)**

(72) Inventors:
• **Kwak, Sung Gun**
**38882 Yeongcheon-si (KR)**
• **Kwac, Da Eun**
**48065 Busan (KR)**

(74) Representative: **Loo, Chi Ching et al**
**Albright IP Limited**
**County House**
**Bayshill Road**
**Cheltenham, Gloucestershire GL50 3BA (GB)**

(54) **METHOD FOR PREPARING COSMETIC PRODUCT USING DEAD SEA MUD AND MACSUMSUK**

(57) The present invention relates to a method for preparing a cosmetic, which is a method for preparing a cosmetic product using Dead Sea mud and Macsumsuk. The Macsumsuk, the Dead Sea mud, and the herbal medicines are processed and granulated and the natural salt is powdered using Macsumsuk, and then the cosmetic composition is prepared by appropriately mixing them. Therefore, the powder flying, the powder agglomeration, and the usability thereof is greatly improved.

EP 4 134 064 A1

## Description

BACKGROUND OF THE INVENTION

Field of the Invention

The present invention relates to a method for preparing a cosmetic, which is a method for preparing a cosmetic product using Dead Sea mud and Macsumsuk.

Description of the Related Art

Stress is accumulating in our body due to various external environments. The stress due to physical stimulation, fatigue, lack of sleep, and emotional tension disrupts the balance of skin hormones and causes various skin problems such as sebum, acne, and rough skin. In particular, the stratum corneum in the enlarged pores around the cheeks and nose does not perform its function, resulting in parakeratosis, which makes the skin texture rough and easy to look rough due to irregularity.
Cells created in the basal layer of the epidermis are differentiated and come up to the stratum corneum, and fully matured cells lose their nuclei in the stratum corneum and are filled with insoluble proteins to become dead cells, resulting in keratolysis.
However, when the abnormal proliferation of keratinocytes in the hair follicle and parakeratosis, which is a keratinization phenomenon, occur, since the immature cells rise to the stratum corneum and the resistance to external stress such as ultraviolet rays is weak, there is no bonding force between the keratinocytes and the skin becomes tattered immediately, resulting in rough skin texture. In addition, excessive sebum secretion through enlarged pores and blackheads caused by sebum oxidation make the complexion rougher.
Therefore, a simple and effective option care product is needed for pore cleansing, moisturizing, and keratin turnover normalizing effects through sebum adsorption. In addition, there is a growing demand for well-being products for healing that restore the mind and body to a healthy state by relieving stress along with resolving skin concerns.
Among the rapidly growing esthetic and spa-related products, packs and masks made of natural materials are the most effective formulations in terms of immediate effectiveness.
However, the existing pack products simply emphasize the origin and rarity of the materials used and are prescribed only with the fine mineral powder itself. Therefore, when using, the powder is blown away and it is difficult to agglomerate or mix when used after being mixed with water or emulsion.
Moreover, it is difficult to control the amount of water or emulsion to be added because there is no viscosity of the mixed contents and it is inconvenient to use because of dripping phenomenon etc. when applied to the face. In addition, there is severe skin tightness when drying, and it is not easy to wash off after drying, and there are cases where the skin feels rather dry after washing.
As a related art, in "Cosmetic composition comprising minerals and plant extracts and method for preparing the same" of Korean Patent Publication No. 10-2006-0080376 (hereinafter referred to as "Patent Literature 1"), a technique for preparing a mask pack by preparing a mineral solution and a plant extract and mixing them in an appropriate ratio is disclosed.

Patent Literature

Patent Literature 1: Korean Patent Publication No. 10-2006-0080376 (July 10, 2006)

SUMMARY OF THE INVENTION

The present invention is to provide a cosmetic composition in a pack formulation, which is prepared by blending and granulating powder containing useful skin ingredients with Macsumsuk salt, herbal medicine extracts, natural preservatives and natural moisturizers, fragrances, pack base materials and the like, and thus exhibits improved usability.
The present invention is to provide a cosmetic composition in a mask pack formulation, which has improved powder flying and powder agglomeration, a soft texture without causing skin tightening during drying, and differentiated appearance.
The present invention is to provide a cosmetic composition in a pack formulation, which is prepared by further blending Macsumsuk salt, GABA (piperidinic acid), and papain of a natural enzyme for exfoliation, and thus has an enhanced effect of improving complexion.
According to an aspect of the invention to achieve the object described above, there is provided a method for preparing a cosmetic product using Dead Sea mud and Macsumsuk, the method including: preparing Macsumsuk granules by heating, grinding, and granulating Macsumsuk raw ore; preparing Macsumsuk salt by washing natural salt, removing impurities from the washed natural salt, and then heating the natural salt in a Macsumsuk furnace; preparing herbal medicine extract granules by processing a herbal medicine extract; preparing Dead Sea mud granules by heating, grinding, and granulating Dead Sea mud; and preparing an additive and then preparing a cosmetic composition by mixing the Macsumsuk granules, the Macsumsuk salt, the herbal medicine extract granules, the Dead Sea mud granules, and the additive together.

In the above configuration, the additive includes any one selected from a natural preservative, a natural moisturizer, a fragrance, or a base material for mask pack.

In addition, the herbal medicine includes any one selected from *Glycyrrhiza uralensis* root extract, *Sophora flavescens* root extract, *Angelica dahurica* root extract, *Liriope platyphylla* root extract, *Morus alba* bark extract, *Acanthopanax sessiliflorus* Seem bark extract, *Paeonia* root extract, *Rehmannia glutinosa* root extract, *Angelica gigas* root extract, *Ligusticum officinale* root extract, *Pleuropterus multiflorus TURCZ.* root extract, *Astragalus propinquus* root extract, *Chrysanthemum indicum* extract, black soybean extract, *Pinus thunbergii* leaf extract, *Lycium chinense* extract, *Chrysanthemum zawadskii* var. *latilobum* extract, deer antler extract, green tea extract, peppermint extract, *Porica* cocas extract, *Saururus chinensis* extract, *Acorus gramineus Solander* extract, *Perilla frutescens var acuta KUDO* leaf water, *Perilla frutescens var acuta KUDO* leaf extract, *Portulaca oleracea* extract, *Artemisia princeps* leaf extract, aloe vera leaf powder, *Houttuynia cordata* extract, *Panax ginseng* extract, *Platycladus orientalis* leaf extract, *Eclipta prostrata* extract, walnut extract, *Nypa fruticans* extract, propolis, *Sciadopitys verticillate, Asteraceae (Carduus crispus),* marigold, *Saururus chinensis,* parsley, *Chrysanthemum coronarium, Prunus mume, Schisandra chinensis, cortex mori radicis, Scutellaria baicalensis, Artemisia campestris,* Sajabalssuk *(Artemisia spp.),* red pine *(Pinus densiflora), Aeschynomene indica, Persicaria perfoliata, Curcuma aromatica, Rheum undulatum, Rumex acetosella, Jeffersonia dubia, Persicaria hydropiper, nemarrhena asphodeloides, Sophora flavescens, Zanthoxylum schinifolium, Rhus javanica,* grapefruit extract, thyme, lemongrass, tea tree, chamomile, manuka, *Eucalyptus globulus, Stevia rebaudiana,* rosemary, lavender, peppermint, *Hippophae rhamnoides,* lime, or lemon.

In addition, the Macsumsuk granules are contained at 30 to 90 wt% and the herbal medicine extract granules are contained at 0.1 to 50 wt% in the mixing step.

In addition, the additive is contained at 0.1 to 20 wt% as a vegetable oligosaccharide, the Macsumsuk granules are contained at 30 to 80 wt%, and the herbal medicine extract granules are contained at 0.1 to 40 wt% in the mixing step.

BRIEF DESCRIPTION OF THE DRAWINGS

The above and other objects, features and advantages of the present invention will be more apparent from the following detailed description taken in conjunction with the accompanying drawings, in which:

FIG. 1 is a graph illustrating an emissivity of far-infrared radiation of Macsumsuk which is used in the present invention;
FIG. 2 to FIG. 4 are micrographs illustrating Macsumsuk granules;

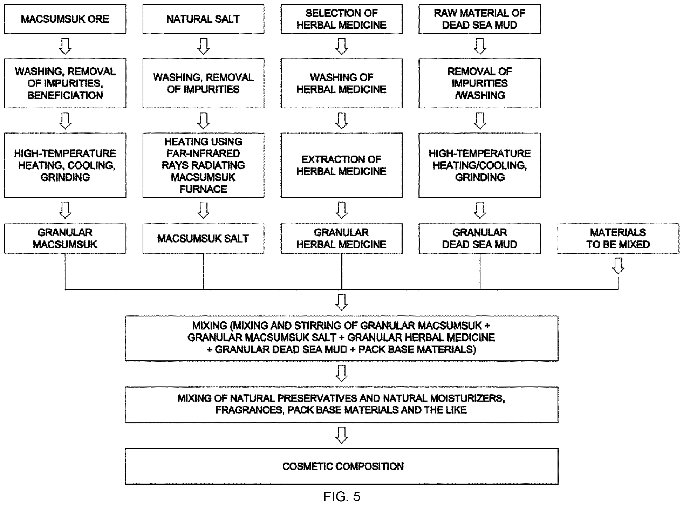

FIG. 5 is a process diagram schematically illustrating the process of the present invention;
FIG. 6 is a test result table illustrating the far-infrared emission amount of Macsumsuk salt, which is a raw material of the present invention; and

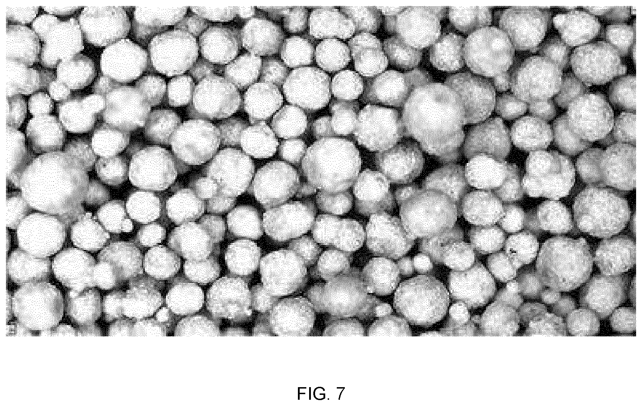

FIG. 7 is an enlarged photograph illustrating the cosmetic composition prepared according to the present invention.

DETAILED DESCRIPTION OF THE PREFERRED EMBODIMENT

Hereinafter, the present invention will be described in detail.

First, Macsumsuk is a kind of ceramics belonging to the monoclinic system, and has white or grayish white pearl luster. The chemical composition of Macsumsuk is based on potassium atom and oxygen atom (oxide), Al and Si atoms, other metal atoms, and hydrogen atoms are bonded to potassium atom and oxygen atom to form an extremely stable crystal structure, and Macsumsuk has been studied as an unspecified crystal formed by irregular substitution of oxygen and potassium atoms in this crystal structure.

Macsumsuk is a natural inorganic material, in which such a crystal is arranged in an orderly manner as a structure shell, and has a hardness of about 2.1 to 2.6, and is thus soft. The whiteness index of Macsumsuk is higher as the purity is higher and the bulky form generally has a higher purity than natural powder.

The components of Macsumsuk used in the present invention are as presented in Table 1.

[Table 1]

| Chemical compound | Content (wt%) |
| --- | --- |
| $SiO_2$ | 64.55 |

(continued)

| Chemical compound | Content (wt%) |
|---|---|
| $Al_2O_3$ | 10.30 |
| CaO | 5.20 |
| MgO | 3.76 |
| $TiO_2$ | 3.21 |
| $ZrO_2$ | 1.85 |
| $Na_2O$ | 1.15 |
| $K_2O$ | 0.77 |
| $Fe_2O_3$ | 1.62 |
| $P_2O_5$ | 0.05 |
| MnO | 0.02 |
| $BaO_6Fe_2O_3$ | 1.55 |
| IgLoss | 5.97 |
| Sum | 100.00 |

Table 2 shows the weight percentage of minerals eluted 3 hours after 1 g of Macsumsuk powder was added to deionized water in a 350 mL container.

[Table 2]

| Amount of powder added (1 g) | | Ca | Mg | Na | k | Fe | Cu | Ar | Cd | Pb |
|---|---|---|---|---|---|---|---|---|---|---|
| Deionized water | | 0.10 | - | - | - | - | - | - | - | - |
| Wt% | 0.5 | 4.92 | 0.60 | 1.05 | 1.52 | 0.52 | - | - | - | - |
| | 1 | 5.65 | 0.83 | 2.37 | 2.81 | 0.78 | - | - | - | - |
| | 1.5 | 5.79 | 1.06 | 3.28 | 3.88 | 1.06 | - | - | - | - |
| | 2 | 6.18 | 1.18 | 4.78 | 4.08 | 1.39 | - | - | - | - |

Table 3 shows the experimental results on the heavy metal removing effect by Macsumsuk.

[Table 3]

| Division of measurement section (h)/unit (number of ions) | $Pb^{2+}$ | $Hg^{2+}$ | $Cd^{2+}$ | $Zn^{2+}$ | $Cu^{2+}$ |
|---|---|---|---|---|---|
| 0.0 | 5,000 | 5,000 | 5,000 | 5,000 | 5,000 |
| 2.0 | 2,650 | 3,250 | 3,520 | 3,390 | 3,830 |
| 4.0 | 1,450 | 2,640 | 2,980 | 2,630 | 3,260 |
| 6.0 | 720 | 2,130 | 2,649 | 1,970 | 2,750 |
| 8.0 | 692 | 1,632 | 2,580 | 1,934 | 2,693 |
| 10.0 | 676 | 1,581 | 2,540 | 1,903 | 2,652 |
| 12.0 | 657 | 1,552 | 2,500 | 1,872 | 2,610 |
| 14.0 | 641 | 1,522 | 2,470 | 1,860 | 2,586 |
| 16.0 | 638 | 1,490 | 2,450 | 1,840 | 2,562 |
| 18.0 | 631 | 1,479 | 2,435 | 1.825 | 2,530 |

(continued)

| Division of measurement section (h)/unit (number of ions) | $Pb^{2+}$ | $Hg^{2+}$ | $Cd^{2+}$ | $Zn^{2+}$ | $Cu^{2+}$ |
|---|---|---|---|---|---|
| 20.0 | 625 | 1,462 | 2,420 | 1.812 | 2,510 |
| 22.0 | 622 | 1,455 | 2,410 | 1.810 | 2,500 |

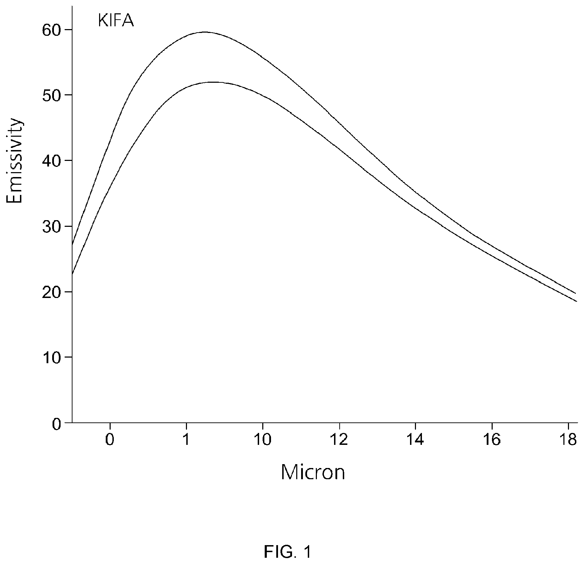

The quantity of far-infrared rays radiated from Macsumsuk used in the present invention is as illustrated in FIG. 1.

1. Preparation of Macsumsuk granules

Macsumsuk mineral is a small particle mineral having a particle size of 0.002 mm or less. The surface area of 1 g of fine particles is as wide as the area of a volleyball court. Macsumsuk mineral exhibits excellent binding properties and thus has an increased hardness when granulated. Macsumsuk mineral has an excellent cation exchange capacity (CFC), thus serves as a buffering agent to have a wet dropping preventing effect, adsorbability, and an effect of removing toxins in the skin, and also functions as a binder in the granule preparing process. In particular, Macsumsuk mineral is known to radiate far-infrared rays in a wavelength band beneficial to the living body, and to have functions of adsorbing impurities by porosity, precipitating minerals, adjusting water quality, and increasing dissolved oxygen in water.

The applicants of the present invention have applied for and registered a method for preparing granular Macsumsuk by utilizing the beneficial functions of Macsumsuk (Korean Patent Publication No. 10-0573811, Patent Document 1). The technology of Patent Document 1 proposes a process of obtaining a slurry by mixing Macsumsuk with water, mixing calcium carbonate with this mixture then adding the resulting mixture into a granulator, and then firing the prepared granules. Meanwhile, as the bubbles inside the granules escape to the outside of the granules in a short time during the molding and firing of granules because of the high temperature, the granules take an annular shape in which a groove is formed on one side as illustrated in the following photograph.

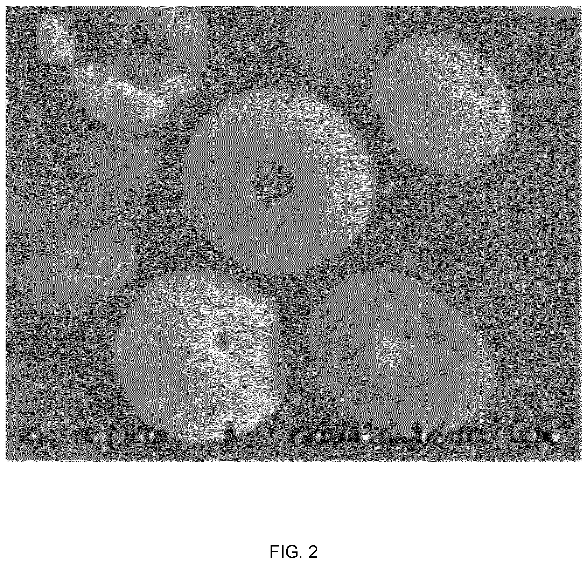

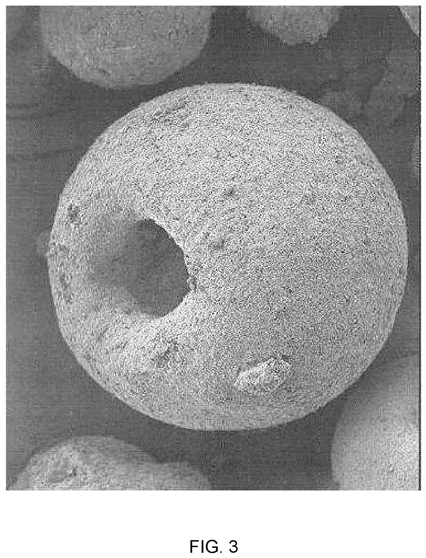

The thus-prepared Macsumsuk granules are illustrated in FIGS. 2 and 3.

When the granules are used as a composition for general pack, such an annular shape with a groove does not cause any problems, but the granules rather serve as a suitable carrier for adsorption and removal of harmful microorganisms propagating on the skin as described in Patent Document 1, and it is thus possible to provide a high-quality pack.

Focusing on the fact that the emissivity of far-infrared rays when Macsumsuk is finely powdered (1 to 6 μm) is higher than the emissivity of far-infrared rays when Macsumsuk in a rocky state, Macsumsuk is powdered and formed into granules in the present invention. The specific method for preparing the Macsumsuk granules is as follows.

a) Preparation of slurry

The slurry is prepared through a supplying step of preparing Macsumsuk raw ore; a heating and cooling step of heating the raw ore at 1,000°C to 1,200°C and then cooling the heated raw ore; a grinding step of grinding the cooled raw ore to have an average particle size in a range of 30 to 40 μm; and a mixing step of mixing the ground Macsumsuk at room temperature for 30 to 50 hours using a ball mill so that the solid powder has a weight ratio of 1:0.2 to 2.0 to prepare a slurry.

b) Preparation of granule mixture

A blowing agent powder selected from the group consisting of calcium carbonate ($CaCO_3$), sodium carbonate ($Na_2CO3$), and any mixture thereof is mixed with the slurry at 0.01 to 0.1 part by weight based on 100 parts by weight of the slurry of Macsumsuk powder to prepare a granule mixture in the form of fluid colloids.

At this time, one to three kinds selected from a dispersant, a pH adjusting agent, or a defoaming agent may be added, and it is preferable that the granule mixture in the form of fluid colloids has a concentration of 30 to 50 wt%, a pH of 7.0 to 9.5, and a viscosity of 300 to 1,550 cps.

c) Preparation of porous granules

Porous granules are prepared through a molding step of spraying the granule mixture into a granulator having an internal temperature of 180°C to 400°C through a nozzle at an injection pressure of 7.0 to 13.0 kg/cm$^2$ to prepare porous granules having an average particle size of 0.1 to 3 mm; and a cooling step of cooling the molded granules.

In this process, calcium carbonate or sodium carbonate mixed as the blowing agent generates carbon dioxide ($CO_2$) by thermal decomposition, carbon dioxide generated inside the granules forms pores inside the granules, the gas is then released to between the granular particles to increase the porosity, and carbon dioxide formed on the surface of the granules increases the surface area of the granules to improve the physical properties of the material.

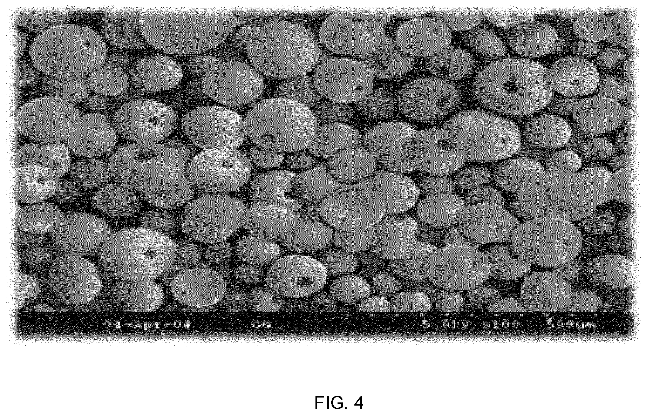

The prepared porous granules are illustrated in FIG. 4.

It is preferable that the prepared porous Macsumsuk granules have a density of 7.0 to 1.22 g/cm$^3$, a specific surface area of 25 to 65 m$^2$/g, a porosity of 47% to 62%, and a size of about 80 to 900 μm.

A plurality of functional clay minerals such as zeolite, bentonite, ZIRPAX, and $TiO_2$ are prepared and each ground and fired to prepare a powder mixture, and this powder mixture may be added in the molding step.

2. Preparation of Dead Sea mud granules

Unlike its name, the Dead Sea, called the Sea of Death, contains minerals in an amount to be 10 or more times the amount of minerals contained in ordinary seawater on average.

For this reason, the mud of the Dead Sea is famous not only for skin beauty but also for muscle pain and arthritis.

The contents of components contained in the mud of the Dead Sea are as presented in Table 4 below.

[Table 4]

| Component | Content (wt%) |
|---|---|
| $Al_2O_3$ | 8.44 |
| BaO | 0.11 |
| CaO | 27.1 |
| $Cr_2O_3$ | 0.03 |
| CuO | 0.01 |
| $Fe_2O_3$ | 5.04 |
| $K_2O$ | 1.43 |
| MgO | 4.26 |
| MnO | 0.05 |
| $Na_2O$ | 0.65 |
| $P_2O_5$ | 1.01 |
| $SiO_2$ | 23.05 |
| $TiO_2$ | 0.42 |
| Ignition loss | 28.4 |
| Sum | 100.0 |

From Table 4 above, it can be seen that $SiO_2$ that has an effect of removing waste and excess sebum from the pores is contained at 23.05 wt% and $Al_2O_3$ that is a main raw material of cosmetics, promotes blood circulation, promotes metabolism, removes waste, and makes the skin clear and transparent is contained at 8.44 wt%.

It can be seen that $Fe_2O_3$ that acts on collagen binding is contained at 5.04 wt% and CaO that is effective in detoxification and stress suppression is contained in a large amount of 27.1 wt%.

The content of MgO that promotes waste excretion and acts on collagen binding is 4.28 wt%, and $K_2O$ that controls osmotic pressure and has a moisture balance controlling and soothing effect is contained at 1.43 wt%.

Next, it can be seen that the content of $Na_2O$ that is a component having an excellent moisture balance controlling and soothing effect important for the skin is 0.65 wt%.

The Dead Sea mud granules may be prepared in the same manner as the Macsumsuk granules, and the temperature may be adjusted.

3. Preparation of Macsumsuk salt

Macsumsuk salt is prepared through:

a) a washing step of removing impurities from the Macsumsuk raw ore in a washing machine;

b) a classification step of adjusting the particle size of the washed raw material to a predetermined particle size or less;

c) centrifugation step of removing heavy sands from the raw material classified in the classification step;

d) a precipitation step of removing moisture from the raw material from which heavy sands have been removed;

e) Macsumsuk powder preparing step of finely powdering and drying the raw material precipitated in the precipitation step to prepare Macsumsuk powder;

f) Macsumsuk ball preparing step of mixing calcined silicon dioxide and platinum with the Macsumsuk powder obtained in the Macsumsuk powder preparing step, then molding the mixture into balls, and firing the balls to form Macsumsuk balls;

g) a Macsumsuk container preparing step of preparing a Macsumsuk container using the Macsumsuk powder obtained in the Macsumsuk powder preparing step;

h) a Macsumsuk furnace preparing step of preparing a Macsumsuk furnace using the Macsumsuk powder obtained in the Macsumsuk powder preparing step;

i) a primary heat treatment step of placing sun-dried salt (salt) to be processed in the Macsumsuk container, then placing the Macsumsuk container in the Macsumsuk furnace, and heating the Macsumsuk container at a temperature of 830°C to 880°C for 8 to 11 hours to remove impurities such as dioxins and heavy metals;

j) a secondary heat treatment step of placing the first heat-treated salt in the Macsumsuk furnace together with Macsumsuk balls containing calcined silicon dioxide and platinum and heating the mixture at a temperature of 830°C to 880°C for 4 to 6 hours to remove dioxins, heavy metals, and impurities;

k) a crystallization step of maintaining the temperature of the Macsumsuk furnace at 330°C to 360°C for 1 to 3 hours and then maintaining the temperature of the Macsumsuk furnace at room temperature for 5 to 7 hours to crystallize the salt; and

l) a powderization step of placing the crystallized salt in a grinder, grinding the crystallized salt, and removing the Macsumsuk balls to prepare the salt into salt powder.

In the configuration, it is preferable that the size of Macsumsuk balls is 10 to 30 mm as a diameter.

The mixing ratio of the sun-dried salt to the Macsumsuk balls is preferably 3.0 to 4.0:1 as a weight ratio.

The fine powderization in the Macsumsuk powder preparing step may be performed by powdering the raw material classified in the classification step by hydrothermal decomposition in a state in which the raw material is mixed with water after the classification step.

In the crystallization step, the temperature of the Macsumsuk furnace may be maintained at 330°C to 360°C for 1 to 3 hours and then cooled to a temperature of 140°C to 150°C, gluconic acid may be mixed with the salt in the Macsumsuk furnace in an amount of 5 to 15 parts by weight based on 100 parts by weight of the salt, and the temperature of the Macsumsuk furnace may be maintained at room temperature for 5 to 7 hours to crystallize the salt.

Alternatively, in the crystallization step, the temperature of the Macsumsuk furnace may be maintained at 330°C to 360°C for 1 to 3 hours and then cooled to a temperature of 60°C to 70°C, a natural extract composed of any one or a mixture of two or more selected from the group consisting of extracts of *Undaria pinnatifida, Saccharina japonica, Cordyceps militaris,* green tea, herbs, and pine needles may be mixed with the salt in the Macsumsuk furnace in an amount of 5 to 25 parts by weight based on 100 parts by weight of the salt, and the temperature of the Macsumsuk furnace may be maintained at room temperature for 5 to 7 hours to crystallize the salt.

Alternatively, in the crystallization step, the temperature of the Macsumsuk furnace may be maintained at 330°C to 360°C for 1 to 3 hours and then cooled to a temperature of 140°C to 150°C, gluconic acid may be mixed with the salt in the Macsumsuk furnace in an amount of 5 to 15 parts by weight based on 100 parts by weight of the salt, the temperature of the Macsumsuk furnace may be cooled to a temperature of 60°C to 70°C, a natural extract composed of any one or a mixture of two or more selected from the group consisting of extracts of *Undaria pinnatifida, Saccharina japonica, Cordyceps militaris,* green tea, herbs, and pine needles may be mixed with the salt in the Macsumsuk furnace in an amount of 5 to 25 parts by weight based on 100 parts by weight of the salt, and the temperature of the Macsumsuk furnace may be maintained at room temperature for 5 to 7 hours to crystallize the salt.

The components of Macsumsuk salt prepared by the present invention and the components of the sun-dried salt before being prepared into Macsumsuk salt are presented in Table 5 for comparison.

[Table 5]

| Component | Common salt | Macsumsuk salt | Analysis |
|---|---|---|---|
| Chloride ion (wt%) | 1.030 | 2.195 | ICP method |
| Sodium (wt%) | 0.131 | 1.032 | |
| Magnesium (wt%) | 441 | 446 | |
| Calcium (mg/L) | 399 | 403 | |
| Potassium (mg/L) | 68.1 | 68.3 | |
| Silicon (mg/L) | 7.61 | 7.64 | |
| Strontium (mg/L) | 4.48 | 4.46 | |
| Boron (mg/L) | 0.025 | 0.031 | |
| Barium (mg/L) | 0.56 | 0.54 | |
| Fluorine (mg/L) | 2627 | 2732 | |
| Sulfate ion ($SO_4$) (mg/L) | 0.03 | 0.02 | |
| Lead ($\mu$g/L) | 0.087 | 0.098 | |
| Cadmium ($\mu$g/L) | 0.008 | 0.011 | |
| Copper ($\mu$g/L) | 0.272 | 0.172 | |
| Iron ($\mu$g/L) | 0.355 | 0.265 | |
| Manganese ($\mu$g/L) | 0.313 | 0.285 | |
| Nickel ($\mu$g/L) | 0.496 | 0.399 | |
| Zinc ($\mu$g/L) | 0.452 | 0.545 | |
| Arsenic ($\mu$g/L) | 0.449 | 0.751 | |
| Molybdenum ($\mu$g/L) | 5.555 | 5.325 | |

The results acquired by analyzing the toxicity and impurities of the prepared Macsumsuk salt are presented in Table 6.

[Table 6]

| Component | Test result | Regulation | Analysis |
|---|---|---|---|
| Moisture(%) | 0.8 | 4.0 or less | Atmospheric pressure drying |
| Arsenic (mg/kg) | Not detected | 0.5 or less | ICP method |
| Lead (mg/kg) | 0.53 | 2.0 or less | |
| Mercury (mg/kg) | 0.008 | 0.1 or less | |
| Sulfate ion (%) | 1.1 | 1.5 or less | ICP method |
| Cadmium (mg/kg) | Not detected | 0.5 or less | |
| Sodium Chloride (%) | 92.3 | 88.0 or more | |
| Total chlorine (%) | 59.3 | 50.0 or more | |
| Insoluble components (%) | 0.7 | 3.0 or less | |
| Sands (%) | Not detected | 0.1 or less | |

The mineral content per 100 g of the prepared Macsumsuk salt and the mineral content per 100 g of commercially available sun-dried salt are presented in Table 7 for comparison.

[Table 7]

| Component name | NaCl | Ca | Mg | K |
| --- | --- | --- | --- | --- |
| Sun-dried salt | 92.9 | 0.03 | 0.34 | 0.01 |
| Table salt | 99.4 | 0.04 | 0.02 | 0.09 |
| Macsumsuk salt | 92.3 | 0.54 | 0.39 | 0.10 |

Table 8 is a table for comparing the content of amino acids per 100 g of prepared Macsumsuk salt and the content of amino acids per 100 g of commercially available sun-dried salt.

[Table 8]

| Amino acid (mg/100g) | Sun-dried salt | Table salt | Macsumsuk salt |
| --- | --- | --- | --- |
| Arginine (mg) | 0 | 0 | 11 |
| Valine (mg) | 0 | 0 | 13 |
| Isoleucine (mg) | 0 | 0 | 7 |
| Lysine (mg) | 0 | 0 | 6 |
| Histidine (mg) | 0 | 0 | 11 |
| Methionine (mg) | 0 | 0 | 6 |
| Phenylalanine (mg) | 0 | 0 | 13 |
| Serine (mg) | 0 | 0 | 10 |
| Leucine (mg) | 0 | 0 | 15 |
| Glutamic acid (mg) | 0 | 0 | 22 |
| Alanine (mg) | 0 | 0 | 1 |

According to the tables, the prepared Macsumsuk salt is rich in minerals containing magnesium, potassium, calcium, phosphorus and the like.

By using the Macsumsuk furnace made of Macsumsuk as a container for heat treatment for the preparation of Macsumsuk salt, at the same time as the impurities are removed, the far-infrared energy radiated from Macsumsuk is absorbed by the salt, and thus an effect of incorporating far-infrared radiation properties beneficial to health as well as removing impurities is acquired.

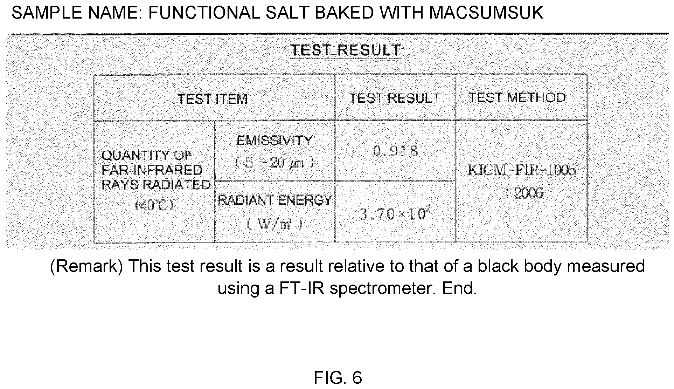

FIG. 6 illustrates the results of a test on the quantity of far-infrared rays radiated from the prepared salt.

4. Preparation of oriental medicine extract granules

a) While a powdery plant, herbal or herbal medicine extract is introduced into a fluidized bed machine and fluidized, the extract powder is sprayed with purified water or a solution of an extract of the same kind as the extract powder as a binder to form microgranules of the extract powder.

The "extract" means a substance separated by extraction from a liquid or solid mixture by an extraction method commonly known in the art, and is in the form of a powder at this stage.

At this time, as the extract, a powder may be purchased or a powdery extract may be directly prepared and used.

Specifically, the dry powder of an extract may be prepared by a drying method commonly known in the art, for example, drying methods such as hot air drying, freeze drying, and natural drying.

The powder of an extract may be prepared using a spray dryer. The preparation of an extract powder by a spray dryer may be carried out under the conditions of an inlet temperature of 145°C to 165°C, an outlet temperature of 75°C to 85°C, and an extract spraying rate of 500 to 1000 ml/hour, but is not limited thereto.

The herbal medicines used in the present invention may be divided into a first oriental medicine biological material, a second oriental medicine biological material, and a third oriental medicine biological material depending on the extraction method.

The first oriental medicine biological material (the one obtained by subjecting a ground material to supercritical extraction using $CO_2$ carrier gas and concentration using a concentrator) includes *Glycyrrhiza uralensis* root, *Sophora*

*flavescens* root, *Angelica dahurica* root, *Liriope platyphylla* root, *Morus alba* bark, *Acanthopanax sessiliflorus* Seem bark, *Paeonia* root, *Rehmannia glutinosa* root, *Angelica gigas* root, *Ligusticum officinale* root, *Pleuropterus multiflorus TURCZ.* Root, and *Astragalus propinquus* root. The second oriental medicine biological material (the one obtained by extracting a ground material using a microwave and a filter) includes *Chrysanthemum indicum,* black soybean, *Pinus thunbergii* leaf, *Lycium chinense, Chrysanthemum zawadskii* var. *latilobum,* deer antler, green tea, peppermint, *Porica* cocas, *Saururus chinensis, Acorus gramineus Solander, Perilla frutescens var acuta KUDO* leaf water, *Perilla frutescens var acuta KUDO* leaf, *Portulaca oleracea, Artemisia princeps* leaf, aloe vera leaf powder, *Houttuynia cordata, Panax ginseng, Platycladus orientalis* leaf, *Eclipta prostrata,* walnut, *Nypa fruticans,* and propolis. The third oriental medicine biological material (the one obtained by subjecting a ground material to supercritical extraction using $CO_2$ carrier gas and concentration using a concentrator at a temperature for a time different from those for the preparation of the first oriental medicine biological material) includes *Sciadopitys verticillate, Asteraceae (Carduus crispus),* marigold, *Saururus chinensis,* parsley, *Chrysanthemum coronarium, Prunus mume, Schisandra chinensis, cortex mori radicis, Scutellaria baicalensis, Artemisia campestris,* Sajabalssuk *(Artemisia spp.),* red pine *(Pinus densiflora), Aeschynomene indica, Persicaria perfoliata, Curcuma aromatica, Rheum undulatum, Rumex acetosella, Jeffersonia dubia, Persicaria hydropiper, nemarrhena asphodeloides, Sophora flavescens, Zanthoxylum schinifolium, Rhus javanica,* grapefruit extract, thyme, lemongrass, tea tree, chamomile, manuka, *Eucalyptus globulus, Stevia rebaudiana,* rosemary, lavender, peppermint, *Hippophae rhamnoides,* lime, and lemon. Herbal medicine containing one or more selected from those listed above as the first, second, and third oriental medicine biological materials may be used.

The binder is sprayed on the seed (powdery extract) and serves to form microgranules of the extract powder through repeated coating and drying or/and serves to form microgranules by binding the extract powder and the extract powder to each other.

The binder is purified water or a solution of an extract (liquid extract), preferably a solution of an extract of the same kind as the extract powder. In the case of using a solution of an extract as a binder, the content of the extract in the microgranules may be increased as compared to the case of using purified water.

The binder may be sprayed from the top of the fluidized bed machine by a top spray method or from the bottom by a bottom spray method, and preferably may be sprayed from the top of the fluidized bed machine by a top spray method. When the binder solution is sprayed while the powder is fluidized, a liquid crosslink is formed between the powder particles. Subsequently, when the moisture evaporates, agglomerates composed of 3 to 4 powder particles are formed by solid crosslinking. After that, when the fine powder particles are continuously bound thereto, so-called raspberry-shaped granules are formed. In the top spray method, while the powder is moving from bottom to top, the binding solution sprayed from the top of the powder movement direction is sprayed from the top to the bottom. Hence, in the top spray method, the opportunity to form liquid crosslinks between the fine particles increases far more than in the bottom spray method because of an increase in the friction force and the contact frequency between particles by the force to move oppositely between the sprayed droplets and the fine particles.

The flow rate, nozzle pressure, hot air temperature, and air volume of the binder may be adjusted in consideration of the viscosity and adhesive strength of the liquid material sprayed as a binder and the moisture evaporation rate of droplets. In other words, when the viscosity and adhesive strength are high, irregular granules are likely to be formed, and it is thus preferable to slow down the flow rate or appropriately dilute the binder. In particular, since moisture easily evaporates in the case of purified water, when the hot air temperature and the air volume are high, the crosslinking force forming action by which fine powder particles are instantaneously dissolved and then bound with each other is lost, and it is difficult to form microgranules.

In step a) of the present invention, the flow rate, nozzle pressure, hot air temperature, and air volume of the binder for forming microgranules of the extract powder may be easily determined by those skilled in the art.

In the case of preparing microgranules by spraying purified water as a binder, the fluidized bed chamber temperature is 70°C to 75°C, the injection rate of the extract is 100 to 250 mL/hour, and the spray pressure may be increased to a range of 0.5 to 5.0 kg/cm$^2$ from 0.5 kg/cm$^2$ of the initial spray pressure while observing the fluidization state. In the case of preparing microgranules by spraying an extract solution as a binder, the fluidized bed chamber temperature is 65°C to 70°C, the injection rate of the extract is 100 to 200 mL/hour, and the spray pressure may be increased to a range of 0.5 to 5.0 kg/cm$^2$ from 0.5 kg/cm$^2$ of the initial spray pressure while observing the fluidization state. According to step a), microgranules of various sizes may be prepared.

b) While the microgranules formed in step a) are introduced into a fluidized bed apparatus as seeds and fluidized, the extract solution is sprayed on the microgranules to grow the microgranules into granules having a predetermined size, whereby microgranules containing an extract at a high concentration are prepared.

5. Mixing of raw materials

The Macsumsuk granules, Dead Sea mud granules, Macsumsuk salt, and oriental medicine extract granules are mixed at a certain ratio.

At this time, the Macsumsuk granules are contained at 30 to 90 wt%, the oriental medicine extract granules are contained at 0.1 to 50 wt%, and the Dead Sea mud granules and the Macsumsuk salt are adjusted in the remaining amount.

In a case where the content of Macsumsuk granules is less than 30 wt%, the viscosity is low and flow is severe when the pack is mixed with water, and there is thus a problem in the pack formulation itself. In a case where the content of Macsumsuk granules exceeds 90 wt%, there is a problem that the pack spreadability is significantly rough and the feel of dryness is severe when the pack is used.

In a case where the content of oriental medicine extract granules is less than 0.1 wt%, there is a problem that the blood circulation improving effect is not significant. In a case where the content of oriental medicine extract granules exceeds 50 wt%, there is a problem that skin safety is diminished.

Specifically, when Macsumsuk granules are contained at 90 wt%, the oriental medicine extract granules may be contained at 0.1 wt%, the Dead Sea mud granules may be contained at 7.9 wt%, and the Macsumsuk salt may be adjusted in the remaining amount.

When the oriental medicine extract granules are contained at 50 wt%, the Macsumsuk granules may be contained at 30 wt%, the Dead Sea mud granules may be contained at 16 wt%, and the Macsumsuk salt may be contained in the remaining amount.

At this time, various additives for imparting functionalities may be added.

For example, GABA (piperidinic acid), a natural enzyme (papain), a vegetable oligosaccharide (raffinose) and the like may be added.

Among these, raffinose, a vegetable oligosaccharide, serves as a binder to bind the Macsumsuk granules, Dead Sea mud granules, Macsumsuk salt, and oriental medicine extract granules to each other.

Raffinose exhibits low hygroscopicity as compared to other sugar components, is thus amorphous, and has an advantage of having an excellent water retention capacity in a low-humidity environment. In the case of granulating the cosmetic composition in a pack formulation by blending raffinose, the granulation yield is improved and the degree of hardening of the prepared granules is optimized to ensure smooth spreadability without stickiness.

Raffinose may be contained at 0.1 to 20 wt% based on the total content, and the remaining amount at this time may be adjusted by Macsumsuk granules or oriental medicine extract granules.

In a case where the content of raffinose is less than 0.1 wt%, there is a problem that granulation and skin improvement are not significant. In a case where the content of raffinose exceeds 20 wt%, there is a problem that the raw material cost is high and the economical efficiency is low.

Hereinafter, Examples of the present invention will be described.

<Example 1>

Macsumsuk raw ore was prepared, heated at 1,100°C, cooled to room temperature, and ground to have an average particle size of 35 mm, the ground material was mixed with water at a weight ratio of 1 : 0.5 at room temperature and the mixture was stirred for 40 hours using a ball mill to prepare a slurry.

Calcium carbonate was prepared as a blowing agent, and mixed with the slurry at 0.05 part by weight based on 100 parts by weight of the slurry to prepare a granule mixture in the form of fluid colloids.

Thereafter, the granule mixture was sprayed into a granulator having an internal temperature of 300°C through a nozzle at an injection pressure of 10.0 kg/cm$^2$ to prepare porous Macsumsuk granules having an average particle size of 0.1 to 3 mm, and the prepared porous Macsumsuk granules were cooled.

Dead Sea mud was prepared, the same process as the process of preparing Macsumsuk granules was performed to prepare Dead Sea mud granules.

Macsumsuk raw ore was washed to remove impurities therefrom, and the washed raw material was classified, heavy sands were removed from the classified raw material, the raw material was precipitated to remove moisture, and the precipitated raw material was powdered and dried to prepare Macsumsuk powder.

Calcined silicon dioxide and platinum were mixed with the Macsumsuk powder, the mixture was molded into balls and fired to prepare Macsumsuk balls having a diameter of 20 mm.

A Macsumsuk container and a Macsumsuk furnace were prepared using the Macsumsuk powder.

Subsequently, sun-dried salt (natural salt) was prepared, placed in the Macsumsuk container, and the Macsumsuk container was placed in the Macsumsuk furnace and heated at a temperature of 830°C to 880°C for 10 hours to remove impurities primarily. The Macsumsuk container from which impurities had been removed was placed in the Macsumsuk furnace together with the Macsumsuk balls and heated at a temperature of 830°C to 880°C for 5 hours to remove impurities secondarily.

At this time, the salt and Macsumsuk balls in the Macsumsuk container were mixed at a weight ratio of 3:1.

Thereafter, the temperature of the Macsumsuk furnace was maintained at 330°C to 360°C for 2 hours, and then maintained at room temperature for 6 hours to crystallize the salt. The crystallized salt was placed in a grinder and ground, Macsumsuk balls were removed, and the salt was powdered to prepare Macsumsuk salt.

*Sciadopitys verticillate, Asteraceae (Carduus crispus),* marigold, *Saururus chinensis,* parsley, *Chrysanthemum coronarium, Prunus mume, Schisandra chinensis, cortex mori radicis, Scutellaria baicalensis, Artemisia campestris,* Sajabalssuk *(Artemisia spp.),* red pine *(Pinus densiflora), Aeschynomene indica, Persicaria perfoliata, Curcuma aromatica, Rheum undulatum, Rumex acetosella, Jeffersonia dubia, Persicaria hydropiper, nemarrhena asphodeloides, Sophora flavescens, Zanthoxylum schinifolium, Rhus javanica,* grapefruit extract, thyme, lemongrass, tea tree, chamomile, manuka, *Eucalyptus globulus, Stevia rebaudiana,* rosemary, lavender, peppermint, *Hippophae rhamnoides,* and lime were all mixed together in the same weight, the mixture was subjected to supercritical extraction using $CO_2$ carrier gas and concentration using a concentrator to prepare a liquid extract, and a part of this liquid extract were dried to prepare a powdery herbal medicine extract.

While the powdery extract was introduced into a fluidized bed apparatus and fluidized, purified water as a binder was sprayed on the extract powder to prepare microgranules of the powder. While the prepared microgranules as seeds were introduced into the fluidized bed machine again, the liquid extract as a binder was sprayed on the microgranules to prepare herbal medicine extract granules.

At this time, the fluidized bed chamber temperature was 70°C, the injection rate of the extract was 150 mL/hour, and the spray pressure was increased to a range of 0.5 to 5.0 kg/cm$^2$ from 0.5 kg/cm$^2$ of the initial spray pressure while observing the fluidization state.

Raffinose was prepared, and a cosmetic composition was prepared by mixing 10 wt% of raffinose, 30 wt% of Macsumsuk granules, 40 wt% of oriental medicine extract granules, 16 wt% of Dead Sea mud granules, and the remaining amount of Macsumsuk salt.

<Example 2>

A cosmetic composition was prepared in the same manner as in Example 1 except that 10 wt% of raffinose, 80 wt% of Macsumsuk granules, 0.1 wt% of oriental medicine extract granules, 7.9 wt% of Dead Sea mud granules, and the remaining amount of Macsumsuk salt were mixed together.

<Example 3>

A cosmetic composition was prepared in the same manner as in Example 1 except that 10 wt% of GABA was added and the content of oriental medicine extract granules was adjusted to 30 wt%.

<Example 4>

A cosmetic composition was prepared in the same manner as in Example 1 except that 3 wt% of papain was added and the content of oriental medicine extract granules was adjusted to 37 wt%.

Hereinafter, Comparative Examples for comparison with Examples will be described.

<Comparative Example 1>

A cosmetic composition was prepared in the same manner as in Example 1 except that Dead Sea mud granules were not contained and the remaining amount of Macsumsuk granules was contained.

<Comparative Example 2>

A cosmetic composition was prepared in the same manner as in Example 1 except that Macsumsuk salt was not contained and the remaining amount of Macsumsuk granules was contained.

<Comparative Example 3>

A cosmetic composition was prepared in the same manner as in Example 1 except that the oriental medicine extract granules were not contained and the remaining amount of Macsumsuk granules was contained.

Hereinafter, Experimental Examples for Examples and Comparative Examples will be described.

<Experimental Example 1> Evaluation on preference

The spreadability, moisturizing feel, skin texture, and pore tightening power of the pack compositions of Examples and Comparative Examples were evaluated.

The evaluation was performed on 25 adults, a 7-point scale method was used for evaluation, and it indicates that the preference is higher as the score is higher.

[Table 9]

| Sample | Spreadability | Moisturizing feel | Skin texture | Pore tightening |
|---|---|---|---|---|
| Example 1 | 5.71±0.25 | 6.86±0.42 | 6.08±0.18 | 6.51±0.76 |
| Example 2 | 5.85±0.12 | 6.75±0.23 | 6.14±0.54 | 6.55±0.48 |
| Example 3 | 5.91±0.46 | 6.65±0.61 | 6.27±0.46 | 6.79±0.12 |
| Example 4 | 5.67±0.36 | 6.25±0.29 | 5.87±0.73 | 6.82±0.32 |
| Comparative Example 1 | 3.22±0.25 | 4.11±0.25 | 4.01±0.44 | 3.22±0.14 |
| Comparative Example 2 | 3.05±0.46 | 3.87±0.41 | 2.97±0.25 | 3.56±0.24 |
| Comparative Example 3 | 2.98±0.22 | 3.34±0.23 | 2.88±0.32 | 3.12±0.41 |

As a result of the evaluation, it has been found that the pack compositions of Examples are remarkably superior to the pack compositions of Comparative Examples in the satisfaction with spreadability, moisturizing feel, skin texture, and pore tightening.

In comparison among Examples, the pack compositions of Examples 2 and 3 are excellent in spreadability, the pack compositions of Examples 1 and 2 are excellent in moisturizing feel, the pack compositions of Examples 2 and 3 are excellent in skin texture, and the pack compositions of Examples 3 and 4 are excellent in pore tightening.

<Experimental Example 2> Measurement of effect of promoting blood circulation and improving complexion

In order to examine whether oriental medicine extract granules promote skin blood circulation, the skin temperature and blood flow rate were measured before and after the actual use of the pack for 20 women aged 25 to 35 years who usually think to have cold hands and feet, divided into two groups.

Thermacam PM575 (FLIR Systems AB, Sweden) was used as a skin temperature measuring instrument, and LDPI (Laser Doppler Perfusion Imager, Perimed AB, Sweden) was used as a blood flow rate measuring instrument.

The subjects washed their face and then acclimatized for 10 minutes in a constant temperature and humidity room (temperature: 21±2°C, relative humidity: 40±2%), and infrared thermal images of the subjects were taken to measure the skin temperature, and blood flow rates at the cheek portion of the subjects were measured.

Subsequently, the packs of Example 1 and Comparative Example 3 were applied to the face of the subjects, dried for 15 minutes, and then washed off, the subjects acclimatized again for 10 minutes in a constant temperature and humidity room, then the measurement was repeated, and the results are presented in Table 10.

[Table 10]

| Sample | | Before application | After application |
|---|---|---|---|
| Example 1 | Blood flow rate (blood flow index; A.U.) | 0.973±0.15 | 1.16±0.17 |
| Comparative Example 3 | | 0.965±0.23 | 0.971 ±0.24 |

As a result of the test, as presented in Table 10, it has been confirmed that the blood flow rate at the cheek portion significantly increases after the use of the pack of Example 1.

As a result of the questionnaire, it has been confirmed that the subjects generally feels to have a smoother skin texture and a brighter complexion.

On the other hand, in the case of Comparative Example 3, it has been confirmed that the increase in blood flow rate is slight.

<Experimental Example 3> Comparison of degree of hardening and physical properties of pack

The cosmetic compositions of Examples and Comparative Examples were each weighed by 8 g, mixed with 12 g of ionized water, applied to a glass plate, and the degree of hardening and physical properties were comparatively evaluated. The results are presented in Table 11 below.

EP 4 134 064 A1

[Table 11]

| Sample | Form of particle | Degree of particle hardening | Compatibility when mixed with water | Spreadability | Scent | Comprehensive evaluation |
|---|---|---|---|---|---|---|
| Example 1 | Granular | Adequate | Normal | Favorable | Good | ○ |
| Example 2 | Granular | Adequate | Excellent | Favorable (slightly grainy) | Good | ○ |
| Example 3 | Granular | Adequate | Excellent | Favorable | Good | ○ |
| Example 4 | Granular | Adequate | Excellent | Favorable | Good | ○ |
| Comparative Example 1 | Irregular (powder) | Low | Poor (powder flying) | Poor (sticking) | Good | × |
| Comparative Example 2 | Granular | Low | Poor (agglomerating) | Poor (flowing) | Normal | × |
| Comparative Example 3 | Irregular (powder) | Low | Normal | Poor (sticking) | Bad | × |
| (○: excellent, △: normal, ×: poor) | | | | | | |

As presented in Table 11, it can be seen that the cosmetic compositions of Examples have an adequate degree of particle hardening, excellent compatibility when mixed with water, and excellent spreadability as compared to the cosmetic compositions of Comparative Examples.

<Experimental Example 4> Comparison of pack stability

The stability of the pack compositions prepared in Examples and Comparative Examples were evaluated.
As a stability evaluation method, the precipitation phenomenon was observed when 1 month had elapsed after the pack compositions were subjected to separation for 30 minutes using a centrifuge.
As a result, it has been confirmed that particular precipitation is not observed from the pack compositions of Examples with the naked eye whereas precipitation has occurred in the pack compositions of Comparative Examples 1 and 3.

<Experimental Example 5> Comparison of exfoliation

To evaluate the exfoliation effect, a Hilltop Chamber containing 0.4 ml of 10% DHA (dihydroxyacetone) solution was attached to the inner portion of the upper arm for 1 hour and then removed. In order to increase the degree of coloring, the process was repeated after 6 hours, and the colored portion was subjected to the measurement using a colorimeter after 24 hours.
The sample was applied to the colored portion by 5 g and dried for 20 minutes, then the dried sample was removed with running water, and the degree of coloring was measured using a colorimeter. The degrees of coloring before and after application of the sample were compared with each other using the ΔE values acquired using the colorimeter, and it can be said that the sample is more effective in exfoliation as the value is larger.
The ΔE value was acquired by the following equation, and the results are presented in Table 12 below.

$$\Delta E = \sqrt{(L1 - L2)^2 + (a1 - a2)^2 + (b1 - b2)^2}$$

In the equation, L, a, and b are as follows.

L : lightness (100 = white, 0 = black)
a : redness (- = green, + = red)
b : yellowness (- = blue, + = yellow)

14

[Table 12]

| Sample | Exfoliation effect ($\Delta E$) |
|---|---|
| Example 1 | 8.8 |
| Example 2 | 9.1 |
| Example 3 | 11.2 |
| Example 4 | 9.5 |
| Comparative Example 1 | 5.6 |
| Comparative Example 2 | 7.1 |
| Comparative Example 3 | 6.9 |

<Experimental Example 6> Comparison of sensory evaluation

The products of Comparative Examples and Examples were applied to the faces of 50 women aged 20 to 35 years according to common usage, the feel of use such as skin feel, moisturized feel, and glossiness after drying was evaluated, and the results are presented in Table 13 below.

The evaluation score was 5 for significantly satisfactory, 4 for satisfactory, 3 for normal, 2 for poor, and 1 for significantly poor, and the composite score was calculated by Equation 1 below.

Composite score = {(number of subjects judged as significantly satisfactory) $\times$ 5 + (number of subjects judged as satisfactory) $\times$ 4 + (number of subjects judged as normal) $\times$ 3 + (number of subjects judged as poor) $\times$ 2 + (number of subjects judged as significantly poor) $\times$ 1} - number of subjects

[Table 13]

| Sample | Skin feel after application and drying | Moisturized feel after application and drying | Glossiness after application and drying |
|---|---|---|---|
| Example 1 | 3.5 | 4.2 | 3.9 |
| Example 2 | 3.7 | 3.9 | 3.8 |
| Example 3 | 3.6 | 4.3 | 3.7 |
| Example 4 | 3.8 | 4.2 | 3.8 |
| Comparative Example 1 | 3.3 | 3.5 | 3.6 |
| Comparative Example 2 | 3.2 | 3.3 | 3.6 |
| Comparative Example 3 | 2.9 | 3.1 | 3.1 |

As presented in Table 13, the preference for the products of Examples is superior to that for the products of Comparative Examples.

The present invention is applicable to various cosmetic compositions, and is more suitably applied to a mask pack.

According to the present invention, the pack composition contains a large amount of organic substances, inorganic substances and the like, and it is thus possible to effectively remove waste and sebum from the pores by osmotic action, have an effect of tightening pores, supplying moisture to the skin, and imparting elasticity to the skin, suppress skin inflammation.

The pack composition has excellent far-infrared radiating function and antibacterial ability and is thus greatly effective in the treatment of skin diseases as well as in blood circulation, metabolism promotion, anti-aging and whitening.

The present invention is used by being mixed with water, emulsion and the like, thus does not cause powder flying, is applied to the skin without agglomeration since the granules are easily mixed with water, emulsions and the like, spreads thickly and closely when applied, and does not cause skin tightening when dried.

In the present invention, a granular pack formulation has been developed, which contains one or more skin useful ingredients selected from the group consisting of herbal medicine powder, Macsumsuk granules and the like, is granulated so as to exhibit improved usability, and has an effect of improving complexion through exfoliation and improvement of skin blood circulation.

The cosmetic composition in a pack formulation according to the present invention can be utilized in various ways to develop immediate-acting natural pack products reflecting the consumer's demand for recent well-being and SPA products.

Industrial Applicability

The present invention can be applied to various cosmetic compositions, but is more suitable when applied to a mask pack.

**Claims**

1. A method for preparing a cosmetic product using Dead Sea mud and Macsumsuk, the method comprising:

    preparing Macsumsuk granules by heating, grinding, and granulating Macsumsuk raw ore;
    preparing Macsumsuk salt by washing natural salt, removing impurities from the washed natural salt, and then heating the natural salt in a Macsumsuk furnace;
    preparing herbal medicine extract granules by processing a herbal medicine extract;
    preparing Dead Sea mud granules by heating, grinding, and granulating Dead Sea mud; and
    preparing an additive and then preparing a cosmetic composition by mixing the Macsumsuk granules, the Macsumsuk salt, the herbal medicine extract granules, the Dead Sea mud granules, and the additive together.

2. The method for preparing a cosmetic product using Dead Sea mud and Macsumsuk according to claim 1, wherein the additive includes any one selected from a natural preservative, a natural moisturizer, a fragrance, or a base material for mask pack.

3. The method for preparing a cosmetic product using Dead Sea mud and Macsumsuk according to claim 1, wherein the herbal medicine includes any one selected from *Glycyrrhiza uralensis* root extract, *Sophora flavescens* root extract, *Angelica dahurica* root extract, *Liriope platyphylla* root extract, *Morus alba* bark extract, *Acanthopanax sessiliflorus* Seem bark extract, *Paeonia* root extract, *Rehmannia glutinosa* root extract, *Angelica gigas* root extract, *Ligusticum officinale* root extract, *Pleuropterus multiflorus TURCZ.* root extract, *Astragalus propinquus* root extract, *Chrysanthemum indicum* extract, black soybean extract, *Pinus thunbergii* leaf extract, *Lycium chinense* extract, *Chrysanthemum zawadskii* var. *latilobum* extract, deer antler extract, green tea extract, peppermint extract, *Porica cocas* extract, *Saururus chinensis* extract, *Acorus gramineus Solander* extract, *Perilla frutescens var acuta KUDO* leaf water, *Perilla frutescens var acuta KUDO* leaf extract, *Portulaca oleracea* extract, *Artemisia princeps* leaf extract, aloe vera leaf powder, *Houttuynia cordata* extract, *Panax ginseng* extract, *Platycladus orientalis* leaf extract, *Eclipta prostrata* extract, walnut extract, *Nypa fruticans* extract, propolis, *Sciadopitys verticillate, Asteraceae (Carduus crispus),* marigold, *Saururus chinensis,* parsley, *Chrysanthemum coronarium, Prunus mume, Schisandra chinensis, cortex mori radicis, Scutellaria baicalensis, Artemisia campestris,* Sajabalssuk *(Artemisia spp.),* red pine *(Pinus densiflora), Aeschynomene indica, Persicaria perfoliata, Curcuma aromatica, Rheum undulatum, Rumex acetosella, Jeffersonia dubia, Persicaria hydropiper, nemarrhena asphodeloides, Sophora flavescens, Zanthoxylum schinifolium, Rhus javanica,* grapefruit extract, thyme, lemongrass, tea tree, chamomile, manuka, *Eucalyptus globulus, Stevia rebaudiana,* rosemary, lavender, peppermint, *Hippophae rhamnoides,* lime, or lemon.

4. The method for preparing a cosmetic product using Dead Sea mud and Macsumsuk according to claim 1, wherein the Macsumsuk granules are contained at 30 to 90 wt% and the herbal medicine extract granules are contained at 0.1 to 50 wt% in the mixing step.

5. The method for preparing a cosmetic product using Dead Sea mud and Macsumsuk according to claim 1, wherein the additive is contained at 0.1 to 20 wt% as a vegetable oligosaccharide, the Macsumsuk granules are contained at 30 to 80 wt%, and the herbal medicine extract granules are contained at 0.1 to 40 wt% in the mixing step.

FIG. 1

FIG. 2

FIG. 3

FIG. 4

| MACSUMSUK ORE | NATURAL SALT | SELECTION OF HERBAL MEDICINE | RAW MATERIAL OF DEAD SEA MUD |
|---|---|---|---|
| ⇩ | ⇩ | ⇩ | ⇩ |
| WASHING, REMOVAL OF IMPURITIES, BENEFICIATION | WASHING, REMOVAL OF IMPURITIES | WASHING OF HERBAL MEDICINE | REMOVAL OF IMPURITIES /WASHING |
| ⇩ | ⇩ | ⇩ | ⇩ |
| HIGH-TEMPERATURE HEATING, COOLING, GRINDING | HEATING USING FAR-INFRARED RAYS RADIATING MACSUMSUK FURNACE | EXTRACTION OF HERBAL MEDICINE | HIGH-TEMPERATURE HEATING/COOLING, GRINDING |
| ⇩ | ⇩ | ⇩ | ⇩ |
| GRANULAR MACSUMSUK | MACSUMSUK SALT | GRANULAR HERBAL MEDICINE | GRANULAR DEAD SEA MUD | MATERIALS TO BE MIXED |

⇩

**MIXING (MIXING AND STIRRING OF GRANULAR MACSUMSUK + GRANULAR MACSUMSUK SALT + GRANULAR HERBAL MEDICINE + GRANULAR DEAD SEA MUD + PACK BASE MATERIALS)**

⇩

**MIXING OF NATURAL PRESERVATIVES AND NATURAL MOISTURIZERS, FRAGRANCES, PACK BASE MATERIALS AND THE LIKE**

⇩

**COSMETIC COMPOSITION**

FIG. 5

SAMPLE NAME: FUNCTIONAL SALT BAKED WITH MACSUMSUK

## TEST RESULT

| TEST ITEM | | TEST RESULT | TEST METHOD |
|---|---|---|---|
| QUANTITY OF FAR-INFRARED RAYS RADIATED (40℃) | EMISSIVITY ( 5 ~ 20 $\mu$m ) | 0.918 | KICM-FIR-1005 : 2006 |
| | RADIANT ENERGY ( W/$m^2$ ) | $3.70 \times 10^2$ | |

(Remark) This test result is a result relative to that of a black body measured using a FT-IR spectrometer. End.

FIG. 6

FIG. 7

Europäisches
Patentamt

European
Patent Office

Office européen
des brevets

# EUROPEAN SEARCH REPORT

Application Number

EP 21 19 3322

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DATABASE GNPD [Online] MINTEL; 31 October 2002 (2002-10-31), anonymous: "Mud Lavender Mask", XP055886108, Database accession no. 10120503 * ingredients list; the whole document * ----- | 1-5 | INV. A61K8/02 A61K8/26 A61K8/96 A61K8/9789 A61Q19/00 |
| X | DATABASE GNPD [Online] MINTEL; 15 April 2011 (2011-04-15), anonymous: "Chamomile Mud Mask", XP055886111, Database accession no. 1529857 * ingredients list; the whole document * ----- | 1-5 | |
| X | DATABASE WPI Week 201810 Thomson Scientific, London, GB; AN 2018-04158W XP002805566, & CN 107 485 591 A (BENMI SHANGHAI TECHNOLOGY CO LTD) 19 December 2017 (2017-12-19) * WPI abstract * ----- | 1-5 | TECHNICAL FIELDS SEARCHED (IPC) A61K A61Q |

-/--

The present search report has been drawn up for all claims

| Place of search | Date of completion of the search | Examiner |
|---|---|---|
| Munich | 4 February 2022 | Briand, Benoit |

EPO FORM 1503 03.82 (P04C01)

page 1 of 2

| | Europäisches Patentamt European Patent Office Office européen des brevets | **EUROPEAN SEARCH REPORT** | **Application Number** EP 21 19 3322 |
|---|---|---|---|

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | KIM BYUNG KI ET AL: "Effects of Kaolinite (Macsumsuk) and Herb Mixtures on the Quality and Physicochemical Properties of Pork", HAN-GUG CHUGSAN SIGPUM HAG-HOEJI – KOREAN SOCIETY FOR FOOD SCIENCE OF ANIMAL RESOURCES, HAN-GUG CHUGSAN SIGPUM HAG-HOEJI, KR, vol. 34, no. 3, 30 June 2014 (2014-06-30), pages 395-402, XP053032774, ISSN: 1225-8563 * the whole document * | 1-5 | |
| Y | DATABASE WPI Week 201005 Thomson Scientific, London, GB; AN 2009-R22217 XP002805567, & KR 100 923 579 B1 (MACSUMSUK GEN MEDICAL CO LTD) 27 October 2009 (2009-10-27) * abstract * | 1-5 | |
| A | ZEEV MA'OR ET AL: "Antimicrobial properties of Dead Sea black mineral mud", INTERNATIONAL JOURNAL OF DERMATOLOGY, WILEY-BLACKWELL PUBLISHING LTD, UK, vol. 45, no. 5, 20 July 2005 (2005-07-20), pages 504-511, XP071187340, ISSN: 0011-9059, DOI: 10.1111/J.1365-4632.2005.02621.X * the whole document * | 1-5 | **TECHNICAL FIELDS SEARCHED (IPC)** |

The present search report has been drawn up for all claims

| Place of search | Date of completion of the search | Examiner |
|---|---|---|
| Munich | 4 February 2022 | Briand, Benoit |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 21 19 3322

This annex lists the patent family members relating to the patent documents cited in the above-mentioned European search report.
The members are as contained in the European Patent Office EDP file on
The European Patent Office is in no way liable for these particulars which are merely given for the purpose of information.

04-02-2022

| Patent document cited in search report | | Publication date | Patent family member(s) | Publication date |
|---|---|---|---|---|
| CN 107485591 | A | 19-12-2017 | NONE | |
| KR 100923579 | B1 | 27-10-2009 | NONE | |

For more details about this annex : see Official Journal of the European Patent Office, No. 12/82

EPO FORM P0459

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020060080376

- KR 100573811